(19) Europäisches Patentamt
European Patent Office
Office européen des brevets

(11) **EP 4 212 089 B1**

(12) **EUROPÄISCHE PATENTSCHRIFT**

(45) Veröffentlichungstag und Bekanntmachung des Hinweises auf die Patenterteilung:
**03.12.2025 Patentblatt 2025/49**

(21) Anmeldenummer: **23151371.4**

(22) Anmeldetag: **12.01.2023**

(51) Internationale Patentklassifikation (IPC):
**A61B 3/032** (2006.01)

(52) Gemeinsame Patentklassifikation (CPC):
**A61B 3/032**

(54) **VERFAHREN ZUM ERMITTELN MINDESTENS EINER EIGENSCHAFT VON MINDESTENS EINEM AUGE EINER PERSON**

METHOD FOR DETERMINING AT LEAST ONE PROPERTY OF AT LEAST ONE EYE OF A PERSON

PROCÉDÉ DE DÉTERMINATION D'AU MOINS UNE PROPRIÉTÉ D'AU MOINS UN IL D'UN SUJET

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **18.01.2022 DE 102022101108**

(43) Veröffentlichungstag der Anmeldung:
**19.07.2023 Patentblatt 2023/29**

(73) Patentinhaber: **Fielmann Ventures GmbH 22083 Hamburg (DE)**

(72) Erfinder: **PAULSEN, Björn 25779 Hennstedt (DE)**

(74) Vertreter: **RDL Patentanwälte PartG mbB Senefelderstrasse 26 70176 Stuttgart (DE)**

(56) Entgegenhaltungen:
**US-A1- 2008 018 858     US-A1- 2021 157 168**

- LANGE C ET AL: "Resolving the clinical acuity categories "hand motion" and "counting fingers" using the Freiburg Visual Acuity Test (FrACT)", GRAEFE'S ARCHIVE FOR CLINICAL AND EXPERIMENTAL OPHTHALMOLOGY ; INCORPORATING GERMAN JOURNAL OF OPHTHALMOLOGY, SPRINGER, BERLIN, DE, vol. 247, no. 1, 3 September 2008 (2008-09-03), pages 137 - 142, XP019657709, ISSN: 1435-702X
- BACH M: "THE FREIBURG VISUAL ACUITY TEST-AUTOMATIC MEASUREMENT OF VISUAL ACUITY", OPTOMETRY AND VISION SCIENCE, WILLIAMS AND WILKINS, BALTIMORE, MD, US, vol. 73, no. 1, 1 January 1996 (1996-01-01), pages 49 - 53, XP002058541, ISSN: 1040-5488

**Beschreibung**

Die Erfindung betrifft ein Verfahren zum Ermitteln mindestens einer Eigenschaft von mindestens einem Auge einer Person, ein System zum Ermitteln mindestens einer Eigenschaft von mindestens einem Auge einer Person und eine Applikation für ein Endgerät zum Ermitteln mindestens einer Eigenschaft von mindestens einem Auge einer Person.

Ein Sehtest für eine Person wird üblicherweise von einem Optiker in einem hierfür vorgesehenen Fachgeschäft durchgeführt.

Vor diesem Hintergrund werden ein Verfahren, ein System und eine Applikation mit den Merkmalen der unabhängigen Patentansprüche vorgestellt. Ausführungsformen des Verfahrens, des Systems und der Applikation gehen aus den abhängigen Patentansprüchen hervor.

US 2008/018858 A1 offenbart ein Optotypendarstellungsgerät zum Testen einer visuellen Funktion eines Auges eines Prüflings, wobei: Ausrichtungssensitive Sehzeichen (z.B. Landoltringe) auf einem Bildschirm dargestellt werden ([0018], [0023]); die Sehzeichen unterschiedliche Größen (entsprechend verschiedener Sehschärfen) und Ausrichtungen haben können ([0022], [0023]); die Sehzeichen nacheinander dargestellt werden mit unterschiedlichen Ausrichtungen und Größen ([0022], [0037]); ein Endgerät mit Bildschirm und Recheneinheit verwendet wird ([0018], [0021]).

LANGE C ET AL: "Resolving the clinical acuity categories "hand motion" and "counting fingers" using the Freiburg Visual Acuity Test (FrACT)", GRAEFE'S ARCHIVE FOR CLINICAL AND EXPERIMENTAL OPHTHALMOLOGY; INCORPORATING GERMAN JOURNAL OF OPHTHALMOLOGY, SPRINGER, BERLIN, DE, Bd. 247, Nr. 1, 3. September 2008 (2008-09-03), Seiten 137-142, XP019657709, ISSN: 1435-702X beschreibt einen computergestützten Sehtest (FrACT) mit folgenden Merkmalen: Landoltringe mit zufälliger Ausrichtung der Lücke werden auf einem Monitor dargestellt; die Größe der Optotypen wird adaptiv basierend auf früheren Antworten angepasst; ein maximaler Likelihood-Algorithmus wird zur Schwellenwertschätzung verwendet; vier nicht-schräge Positionen der Lücke (Ausrichtungswinkel) werden verwendet; ein Forced-Choice-Verfahren wird angewendet, bei dem der Patient immer eine Richtung angeben muss.

US 2021/157168 A1 betrifft ein Verfahren zur Optimierung einer optischen Hilfe mittels automatischer Bestimmung der subjektiven Sehschärfe, wobei: Landoltringe als Stimulus-Bilder auf einem Monitor präsentiert werden; die Lücke des Landoltrings verschiedene Ausrichtungen haben kann (acht mögliche Winkel); die Landoltringe mit verschiedenen Bildgrößen präsentiert werden; ein neuronales Netzwerk zur Auswertung genutzt wird; die subjektive Sehschärfenschwelle für verschiedene optische Systeme bestimmt wird.

Das erfindungsgemäße, üblicherweise adaptive und/oder computerimplementierte, Verfahren ist zum Ermitteln mindestens einer üblicherweise optometrischen Eigenschaft von mindestens einem Auge, in der Regel von zwei bzw. beiden Augen oder nur einem Auge, einer Person mit einem Endgerät und somit zum Durchführen eines Sehtests für diese Person vorgesehen. Dabei werden auf und/oder mit einem Bildschirm bzw. einem Display des Endgeräts ausrichtungssensitive Sehzeichen bzw. Optotypen dargestellt, wobei jedes ausrichtungssensitive Sehzeichen bzw. jeder Optotyp eine bspw. geometrische Eigenschaft aufweist, die von einem Ausrichtungswinkel $\omega$ und mindestens einem Markierungsabstand und/oder Geometrieabstand zwischen mindestens jeweils zwei Markierungen als geometrische Eigenschaft des ausrichtungssensitiven Sehzeichens abhängig bzw. gebildet ist, d. h., dass diese mindestens zwei Markierungen, als mindestens zwei Punkte, die ausrichtungswinkelabhängige geometrische Eigenschaft eines jeweiligen Sehzeichens bilden. Weiterhin wird für mindestens drei unterschiedliche Ausrichtungswinkel $\omega$ jeweils eine Schar von ausrichtungssensitiven Sehzeichen ermittelt, wobei die jeweiligen Markierungsabstände der mindestens zwei Markierungen der ausrichtungssensitiven Sehzeichen jeweils einer Schar für einen Ausrichtungswinkel $\omega$ von Sehzeichen zu Sehzeichen unterschiedlich sind , wobei auf dem Bildschirm nacheinander für jeden Ausrichtungswinkel $\omega$ die ausrichtungssensitiven Sehzeichen auf dem Bildschirm dargestellt werden, wobei für einen jeweiligen Ausrichtungswinkel $\omega$ mit einem ersten ausrichtungssensitiven Sehzeichen mit einem definierten jeweiligen Markierungsabstand zwischen jeweils zwei der mindestens zwei Markierungen begonnen wird. Danach werden sukzessiv weitere ausrichtungssensitive Sehzeichen mit jeweils anderen Markierungsabständen zwischen den mindestens zwei Markierungen dargestellt, bei denen der jeweilige Markierungsabstand zwischen den mindestens zwei Markierungen von dem des ersten Sehzeichens abweicht und auch die jeweiligen Markierungsabstände der weiteren Sehzeichen von Sehzeichen zu Sehzeichen voneinander abweichen, wobei der jeweilige Markierungsabstand von Sehzeichen zu Sehzeichen entweder verkleinert oder vergrößert und somit verändert ist. Dabei wird, üblicherweise nach Darstellung mindestens eines weiteren Sehzeichens, bestimmt, welcher minimale Markierungsabstand zwischen den Markierungen für den jeweiligen Ausrichtungswinkel $\omega$ für das mindestens eine Auge und/oder von dem mindestens einen Auge der Person noch erkennbar ist, üblicherweise optisch auflösbar ist. Außerdem wird für jeden Ausrichtungswinkel $\omega$ der minimale Markierungsabstand als Grenzabstand g ermittelt, wobei aus einer Mehrzahl von Grenzabständen eine charakteristische Funktion des mindestens einen Auges und/oder für das mindestens eine Auge abgeleitet wird.

Dabei wird das Verfahren üblicherweise von einer softwaregestützten Applikation bzw. Anwendung, die auf, von und/oder mit dem Endgerät ausgeführt wird, automatisch kontrolliert und somit gesteuert und/oder geregelt. In Ausgestaltung wird der Person für jedes dargestellte Sehzeichen mit einem jeweiligen Markierungsabstand zwischen den mindestens zwei Markierungen, von dem Endgerät ein optisches und/oder akustisches Signal bereitgestellt, das von, auf und/oder mit dem Bildschirm dargestellt und/oder von einem Lautsprecher des Endgeräts ausgegeben wird. Dabei wird die Person mit diesem Signal gefragt, ob sie den jeweiligen Markierungsabstand zwischen den mindestens zwei Markierungen des jeweils aktuell dargestellten n-ten Sehzeichens noch erkennen, bspw. optisch auflösen bzw. trennen, kann oder nicht.

Es ist möglich, dass der Person insgesamt k Sehzeichen nacheinander dargestellt werden, bis jenes ausrichtungssensitive Sehzeichen mit dem minimalen Markierungsabstand zwischen den Markierungen dargestellt und weiterhin ermittelt wird, dass der Markierungsabstand für das mindestens eine Auge und/oder von dem mindestens einen Auge der Person noch erkennbar ist bzw. noch erkannt werden kann. Dabei wird der Person jeweils aktuell ein n-tes Sehzeichen mit einem n-ten Wert für den Markierungsabstand zwischen den Markierungen dargestellt. Dabei sind k und n ganze natürliche Zahlen, wobei n größer oder gleich 1 und kleiner oder gleich k ist. Eine Änderung, also Vergrößerung oder Verkleinerung, zwischen einem n-1-ten Wert des Markierungsabstands eines n-1-ten dargestellten Sehzeichens und dem n-ten Wert des Markierungsabstands des n-ten dargestellten Sehzeichens bzw. zwischen dem n-ten Wert des Markierungsabstands des n-ten dargestellten Sehzeichens und einem n+1-ten Wert des Markierungsabstands eines n+1-ten dargestellten Sehzeichens kann dabei je nach Bedarf, bspw. von der Person und/oder von dem Endgerät bzw. der damit ausgeführten Applikation, verfahrensbegleitend, bspw. automatisch, durch entsprechende Auswahl des bzw. der darzustellenden Sehzeichen als n-1-tes darzustellendes Sehzeichen bzw. als n-tes darzustellendes Sehzeichen eingestellt und/oder angepasst werden.

Falls die Person den Markierungsabstand mit dem n-ten Wert für das aktuelle n-te Sehzeichen noch erkennen kann bzw. hierzu in der Lage ist, wird nachfolgend als n+1-tes Sehzeichen ein Sehzeichen ausgewählt, dessen Markierungsabstand gegenüber dem Markierungsabstand des n-ten Sehzeichens verkleinert bzw. reduziert ist. Falls die Person dies für das aktuelle n-te Sehzeichen dagegen nicht kann bzw. hierzu nicht in der Lage ist, wird nachfolgend als n+1-tes Sehzeichen ein Sehzeichen ausgewählt, dessen Markierungsabstand gegenüber dem Markierungsabstand des n-ten Sehzeichens vergrößert ist. In diesem zweiten Fall wird als n+1-tes Sehzeichen ein Sehzeichen gewählt. dessen Markierungsabstand zwischen den bspw. zwei Markierungen zwar größer als der n-te Wert des Markierungsabstands des n-ten Sehzeichens aber geringer bzw. kleiner als der n-1-te Wert für den Markierungsabstand des n-1-ten Sehzeichens ist. Hierdurch ist es möglich, den für die Person erkennbaren Markierungsabstand iterativ und präzise zu ermitteln.

Es ist möglich, als erstes dargestelltes Sehzeichen ein Sehzeichen mit einem initialen Markierungsabstand in Abhängigkeit von einem Alter der Person und/oder mindestens einer bereits bekannten optometrischen Eigenschaft des mindestens einen Auges der Person zu wählen, wobei die Person ihr Alter und/oder die mindestens eine bekannte optometrische Eigenschaft in das Endgerät eingibt, bevor das erste Sehzeichen dargestellt wird. Bei bzw. mit dem adaptiven Verfahren ist es u. a. möglich, dass der aktuell gezeigte Markierungsabstand bzw. das diesem Markierungsabstand zugeordnete Sehzeichen jeweils von einer Antwort der Person auf den vorherigen Markierungsabstand bzw. den initialen Markierungsabstand mit demselben Ausrichtungswinkel $\omega$ abhängig ist. Ist bspw. der vorherige Markierungsabstand unter diesem Ausrichtungswinkel $\omega$ erkannt worden, wird als nächstes Sehzeichen ein Sehzeichen mit einem kleineren Markierungsabstand präsentiert. Falls nicht, wird als nächstes Sehzeichen ein Sehzeichen mit einem größeren Markierungsabstand gewählt. Hier ist weiterhin möglich, die Sehzeichen sukzessive so zu wählen und darzustellen, dass der dargestellte Markierungsabstand sich linear ändert, bis der Grenzabstand gefunden wird. Ergänzend oder alternativ wird durch geeignete Wahl der sukzessiv darzustellenden Sehzeichen der Markierungsabstand um den Grenzabstand herum in genaueren Schritten durch minimales Vergrößern und Verkleinern variiert bzw. verändert, wobei der Grenzabstand noch genauer bestimmt wird. Eine mögliche optometrische Eigenschaft kann als ein möglicherweise vorhandener Sehfehler des mindestens einen Auges ausgebildet sein bzw. bezeichnet werden, der bei dem Verfahren entdeckt wird.

In Ausgestaltung wird bzw. ist die charakteristische Funktion als Nullstellenfunktion einer Modulationsübertragungsfunktion MTF in einem virtuellen Koordinatensystem ausgebildet bzw. ausgeprägt.

Dabei wird für die charakteristische Funktion eine rotierte bzw. rotierende Ellipse mit einem Fit angepasst, wobei aus mindestens drei ausrichtungswinkelabhängigen Grenzabständen g eine große Halbachse b, eine kleine Halbachse a und ein Rotationswinkel $\phi$ der Ellipse als Ellipsenparameter ermittelt werden. Diese rotierte Ellipse kann auch als primäre Ellipse bezeichnet werden. Die rotierte Ellipse ist eine Möglichkeit der verallgemeinerten charakteristischen Funktion, bspw. eine vorgegebene mathematische Funktion mit unbekannten Parametern, die angefittet bzw. angepasst werden. In der Regel wird die Ellipse angenähert.

Dabei ist es möglich, dass Grenzfrequenzen $f_g$, welche aus den Grenzabständen g mit bzw. unter einem bestimmten Ausrichtungswinkel $\omega$ eines jeweiligen Sehzeichens ermittelt werden, mit einem entsprechenden Achsenwinkel $\alpha$ eines Tabo-Schemas (technischer Ausschuss für Brillenoptik) ein Messwertepaar $(f_g, \phi)$ bilden. Dabei wird jeweils eine ausrichtungswinkelabhängige Grenzfrequenz $f_g$ aus einem Kehrwert eines jeweiligen ausrichtungswinkelabhängigen Grenzabstands g berechnet. An die mindestens drei aufgenommenen Messwertepaare wird die rotierte Ellipse mit Mittelpunkt im Ursprung des Koordinatensystems gefittet, welches durch die Achsenwinkel des TABO-Schemas aufgespannt wird und den Frequenzraum der MTF darstellt. Die Ellipse folgt hierbei der allgemeinen Polar-

Form:

$$r(\phi) = \frac{ab}{\sqrt{(b\cos(\alpha-\phi))^2+(a\sin(\alpha-\phi))^2}},$$

wobei a eine Länge der kleinen Halbachse, b eine Länge der großen Halbachse und $\phi$ den Rotationswinkel der Ellipse darstellen. Hierbei ist es weiterhin möglich, zur Evaluierung der Ellipsenparameter, ein neuronales Netz einzubeziehen und/oder zu verwenden.

Hierzu können experimentelle Daten für Grenzfrequenzen mit den optimalen, aus den möglichen Sehfehlern Sphäre, Zylinder und Achse abgeleiteten Ellipsen von Personen zum Trainieren des neuronalen Netzes verwendet werden.

Als charakteristische Funktion kann neben der Nullstellenfunktion der MTF auch eine konstante Kontrastübertragungsfunktion verwendet werden, die wieder eine Ellipse ist. Diese konstante Kontrastübertragungsfunktion ergibt sich bspw. wenn ein Objektkontrast des Optotypen auf einen konstanten Wert verringert wird. Eine zusätzliche charakteristische Funktion ergibt sich auch durch die Beschreibung des Sehfehlers und somit eines Dioptrienwerts in der Form:

$$\text{Dpt }(\phi) = \text{Sphäre} + \text{Zylinder} * \text{sind }(\phi - \text{Achse})^2$$

Diese charakteristische Funktion kann auch mit Hilfe eines Proportionalitätsfaktors an die Grenzfrequenzen $f_g$ gefittet werden.

Gemäß dem Tabo-Schema werden in Ausgestaltung des Verfahrens für jedes Auge der Person als Sehzeichen Halbkreise oder Landoltringe abgebildet bzw. dargestellt, wobei sich ein Halbkreis von 0° bis 180° erstreckt. Dabei erstreckt sich bspw. der Halbkreis für ein rechtes Auge nasal von 0° gegen den Uhrzeigersinn bis 180°. Für das linke Auge erstreckt sich der Halbkreis nasal von 180° im Uhrzeigersinn bis 0°.

Aus der jeweiligen Ausrichtung des Sehzeichens, bspw. Landoltring oder Gitter, wird der Ausrichtungswinkel $\omega$ bspw. eines jeweiligen Landoltrings oder eines Gitters in einen Winkel, hier den voranstehend eingeführten Achsenwinkel $\alpha$, des Tabo-Schemas bzw. einen sog. Tabowinkel umgerechnet. Die Winkel im Tabo-Schema sind zu den Ausrichtungswinkeln der Optotypen auf dem Bildschirm an einer vertikalen Achse gespiegelt. Der Tabowinkel $\alpha$ ergibt sich aus einem Ausrichtungswinkel $\omega$ durch $\alpha$ = 180° - $\omega$.

Im Fall des Landoltrings sind die mindestens zwei Markierungen, bspw. zwei Markierungen, als geometrische Eigenschaften als Punkte ausgebildet. Dabei liegen die beiden Punkte, die eine Lücke bzw. Öffnung bzw. Ausnehmung des Landoltrings begrenzen und so einen Markierungsabstand zwischen den zwei Markierungen bilden, auf einer Linie, deren Ausrichtung im Koordinatensystem den Ausrichtungswinkel $\omega$ definiert. Falls die Lücke bzw. Öffnung bzw. der Markierungsabstand eines Landoltrings bspw. nach rechts weist bzw. nach rechts oder links bzw. horizontal ausgerichtet ist, liegen die Punkte, die die Lücke bzw. Öffnung begrenzen, auf einer Linie, die senkrecht bzw. vertikal nach oben zeigt bzw. orientiert ist, üblicherweise entlang einer Ordinate bzw. y-Achse, des Koordinatensystems, was einem Ausrichtungs-winkel $\omega$ von 90° bzw. 270° entspricht. Falls die Lücke bspw. nach oben oder unten weist bzw. vertikal ausgerichtet bzw. orientiert ist, ist die Linie, auf der die Punkte, die die Lücke begrenzen, liegen, parallel zur x-Achse bzw. Abszisse des Koordinatensystems bzw. horizontal orientiert und entspricht einem Ausrichtungswinkel $\omega$ von 0° bzw. 180°.

In Ausgestaltung werden aus den Ellipsenparametern der rotierten Ellipse, d. h. aus der großen Halbachse b, der kleinen Halbachse a und dem Rotationswinkel $\phi$, als Geometrieparameter eine primäre Sphäre, ein primärer Zylinder und eine primäre Achse ermittelt, wobei aus mindestens einem Geometrieparameter mindestens ein Dioptrienwert ermittelt wird, und/oder wobei mindestens ein Verhältnis bzw. Ratio aus zwei der Geometrieparameter relativ zueinander ermittelt wird. Dies betrifft bspw. ein Verhältnis der primären Sphäre zu dem primären Zylinder und/oder ein Verhältnis der primären Sphäre und/oder des primären Zylinders zu der primären Achse. Durch die Achse und den Zylinder wird die mindestens eine optometrische Eigenschaft des mindestens einen Auges bestimmt.

Die Sphäre und der Zylinder sind üblicherweise eindimensionale Zahlenwerte, die einerseits den Anteil des rein sphärischen Sehfehlers, also des symmetrischen Sehfehlers relativ zur optischen Achse, und andererseits des zylindrischen Sehfehlers, also des Sehfehlers mit einer Vorzugsrichtung, bspw. einem Meridian, in Dioptrien angeben. Die Ellipse und/oder die Nullstellenfunktion der MTF beschreibt einen Lichtfleck, der durch den Sehfehler verzerrt aus einer Punktlichtquelle auf der Retina eines Auges entsteht. Daher ist die eine Halbachse der Ellipse, also die große oder die kleine, proportional zur Stärke des sphärischen Sehfehlers, also der Sphäre, während die andere Halbachse der Ellipse, also die kleine oder die große, proportional zur Summe des sphärischen und des zylindrischen Sehfehlers ist.

Es ist auch möglich, die Ellipsenparameter direkt aus zylindrischen und/oder sphärischen Achsen des Tabo-Schemas und des Sphäre-Zylinder-Verhältnisses der primären Sphäre zu dem primären Zylinder zu ermitteln. Neben dem Schritt, die primäre Sphäre, den primären Zylinder und die primäre Achse zu bestimmen, ist es auch möglich, dass das Verhältnis aus der primären Sphäre und dem primären Zylinder zusammen mit der primären Achse bestimmt wird. Hierbei ist es möglich, die primäre Sphäre und den primären Zylinder unter Hinzunahme eines weiteren Prozessparameters oder mehrerer weiterer Prozessparameter, z. B. eines Messabstands zwischen dem mindestens einen Auge und dem Bildschirm, auf bzw. mit dem die ausrichtungssensitiven Sehzeichen dargestellt werden, und/oder eine Pupillengröße der Person gesondert zu bestimmen.

In Ausgestaltung werden die primäre Sphäre, der primäre Zylinder und die primäre Achse als Geometrieparameter durch einen Einsatz einer künstlichen Intelligenz, u. a. aus den ausrichtungswinkelabhängigen Grenzabständen g, ermittelt.

In weiterer Ausgestaltung werden durch Einbezug von dem mindestens einen weiteren Prozessparameter aus dem Verhältnis der primären Sphäre und des primären Zylinders eine sekundäre Sphäre und ein sekundärer Zylinder als Dioptrienwerte ermittelt. Dabei ist bzw. sind der hierfür vorgesehene mindestens eine weitere, bspw. prozessbezogene, Parameter bzw. Prozessparameter als der Messabstand des mindestens einen Auges der Person zu dem Bildschirm bzw. des Monitors des Endgeräts und/oder als die Pupillengröße von dem mindestens einen Auge der Person ausgeprägt bzw. ausgebildet.

Der mindestens eine Prozessparameter wird durch eine Sensorik, also durch mindestens einen Sensor, bspw. einen Abstandsmesser und/oder eine Kamera, des Endgeräts gemessen und somit erfasst bzw. ermittelt.

Es ist weiterhin möglich, dass die voranstehend vorgesehene Modulationsübertragungsfunktion MTF durch:

$$\text{MTF(f)} = |\text{PSF(f)}|^2 = \text{Bildkontrast (f)/ Objektkontrast (f)}$$

gebildet wird, wobei PSF eine Punktspreizfunktion ist, und wobei als Ortsfrequenz f und somit als Grenzfrequenz $f_g$ ein ausrichtungswinkelabhängiger Grenzabstand g bzw. dessen Kehrwert verwendet wird. Dabei bezeichnet Objektkontrast den Kontrast des jeweils von dem Bildschirm präsentierten und von der Person betrachteten ausrichtungssensitiven Sehzeichens. Hier kann der Objektkontrast durch die zur Darstellung ausgewählten Pixelwerte definiert werden. Der Bildkontrast bezeichnet den Kontrast eines entstehenden Retinabilds auf der Retina bzw. Netzhaut des mindestens einen Auges der Person. Hierbei werden Nullstellen der MTF ebenfalls durch Abfrage der Person gefunden und/oder ermittelt, wobei die Person gefragt wird, ob von ihr ein jeweiliger Grenzabstand noch identifiziert werden kann oder nicht. Durch zusätzliche Nutzung eines jeweils genannten Kontrasts ist es u. a. möglich, dass der oben genannte Prozess für verschiedene Ausgangs-Objektkontraste durchgeführt wird. Daraus werden weitere Ellipsen ermittelt und/oder erhalten. Dabei ist jeweils eine derartige, üblicherweise sekundäre, Ellipse für einen fixen Objektkontrast, bspw. Ausgangs-Objektkontrast, vorgesehen, wobei diese denselben Rotationswinkel $\phi$ und dasselbe Verhältnis zwischen Längen ihrer beiden Halbachsen a, b wie die voranstehend genannte primäre rotierte Ellipse besitzen bzw. aufweisen sollte. Die Ellipsenparameter der sekundären Ellipse können dann für ein genaueres Ergebnis durch Vergleich oder Kombination verwendet werden.

In weiterer Ausgestaltung des Verfahrens werden als ausrichtungssensitive Sehzeichen Landoltringe verwendet, wobei jeder ausrichtungssensitive Landoltring als geometrische ausrichtungswinkelabhängige Eigenschaft die Öffnung bzw. Lücke aufweist, wobei jede Lücke durch mindestens zwei Punkte als Markierungen begrenzt ist, die Enden des ausrichtungssensitiven Landoltrings bilden, wobei von dem Endgerät für jeden Ausrichtungswinkel $\omega$ eine Schar an ausrichtungssensitiven Landoltringen dargestellt wird, wobei Enden der ausrichtungssensitiven Landoltringe voneinander bzw. zueinander den Markierungsabstand aufweisen. Alternativ ist es möglich, als Optotypen bzw. Sehzeichen Symbole, bspw. Buchstaben bzw. Zahlen, oder Gitter zu verwenden, für die ebenfalls ein Markierungsabstand vorgesehen ist, der für Markierungen entsprechender Symbole ausrichtungswinkelabhängig variiert wird. Die Ausrichtungswinkel $\omega$ werden über ein Intervall [0°, 180°] gleichmäßig verteilt. Dabei kann bspw. mit Ausrichtungswinkeln von 0°, 30°, 60° und 90° eine stabile Verteilung bereitgestellt werden, wobei für jeweils zwei dieser Ausrichtungswinkel $\omega$ bzw. $\omega_{i+1}$, $\omega_i$ definitionsgemäß bspw. $\omega_{i+1} = \omega_i + 30°$ gilt. Bei dem Verfahren können Optotypen mit unterschiedlichen charakteristischen Details und/oder Ausrichtungswinkeln verwendet werden, bspw. ein Optotyp mit kombinierten Gitter- bzw. Grating-Mustern.

Das erfindungsgemäße System ist zum Ermitteln mindestens einer üblicherweise optometrischen Eigenschaft, bspw. eines möglicherweise vorhandenen Sehfehlers, von mindestens einem Auge einer Person und somit zum Durchführen eines Sehtests für diese Person ausgebildet. Das System weist ein Endgerät auf, das einen Bildschirm und eine Recheneinheit aufweist, wobei das Endgerät dazu ausgebildet ist, auf dem Bildschirm ausrichtungssensitive Sehzeichen darzustellen, wobei jedes ausrichtungssensitive Sehzeichen eine geometrische Eigenschaft, üblicherweise mindestens ein Muster, aufweist, die von einem Ausrichtungswinkel $\omega$ und mindestens einem Markierungsabstand zwischen mindestens jeweils zwei Markierungen des ausrichtungssensitiven Sehzeichens abhängig und/oder gebildet ist, wobei das Endgerät dazu ausgebildet ist, für mindestens drei unterschiedliche Ausrichtungswinkel $\omega$ jeweils eine Schar von ausrichtungssensitiven Sehzeichen zu ermitteln, wobei die mindestens zwei Markierungen als Muster der ausrichtungssensitiven Sehzeichen jeweils einer Schar für einen Ausrichtungswinkel $\omega$ von Sehzeichen zu Sehzeichen unterschiedliche Markierungsabstände aufweisen, wobei das Endgerät dazu ausgebildet ist, auf dem Bildschirm nacheinander für jeden Ausrichtungswinkel ω die ausrichtungssensitiven Sehzeichen darzustellen und dabei für einen jeweiligen Ausrichtungswinkel ω mit einem ausrichtungssensitiven Sehzeichen zu beginnen, bei dem der mindestens eine Markierungsabstand zwischen den Markierungen ein üblicherweise definierter, bspw. definierbarer fester, Wert ist, und danach sukzessiv weitere ausrichtungssensitive Sehzeichen mit jeweils mindestens einem anderen Markierungsabstand zwischen den Markierungen darzustellen, wobei der mindestens eine Markierungsabstand zwischen den Markierungen von Sehzeichen zu Sehzeichen zu verändern bzw. veränderbar ist, wobei das Endgerät dazu ausgebildet ist, zu überprüfen, welcher minimale Markierungsabstand zwischen den Markierungen für den jeweiligen Ausrichtungswinkel ω des jeweils dargestellten ausrichtungssensitiven Sehzeichens für die Person noch erkennbar ist, wobei das Endgerät dazu ausgebildet ist, für jeden Ausrichtungswinkel ω den minimalen Markierungsabstand als Grenzabstand g zu ermitteln.

Es ist möglich, eine Ausführungsform des vorgestellten Verfahrens mit einer Ausführungsform des vorgestellten Systems, bspw. eines adaptiven Systems, durchzuführen. Dabei kann eine Größe der Sehzeichen bzw. der von diesen jeweils umfassten Markierungsabständen randomisiert variiert werden. In weiterer Ausgestaltung werden die Ausrichtungswinkel ω der Sehzeichen zwischen den einzelnen dargestellten bzw. präsentierten Sehzeichen variiert, wobei der mindestens eine Markierungsabstand für den jeweiligen Ausrichtungswinkel ω, wie oben beschrieben, adaptiv von der vorherigen Antwort bei diesem Ausrichtungswinkel ω gewählt wird.

Die Recheneinheit des Systems ist dazu ausgebildet, aus entsprechend mindestens drei Grenzabständen g für die mindestens drei unterschiedlichen Ausrichtungswinkel ω als Nullstellen einer Modulationsübertragungsfunktion MTF in einem virtuellen Koordinatensystem die rotierte Ellipse mit einem Fit anzupassen und aus den mindestens drei ausrichtungswinkelabhängigen Grenzabständen g, die große Halbachse b, die kleine Halbachse a und den Rotationswinkel ϕ der Ellipse zu ermitteln.

Das Endgerät ist als mobiles bzw. manuell tragbares Datenverarbeitungs- und Kommunikationsgerät, bspw. als sog. Smartphone, als Laptop, Notebook, als Tablet oder als Personalcomputer ausgebildet und/oder zu bezeichnen, wobei dieses Endgerät von der Person auch für andere Zwecke unabhängig von dem Verfahren genutzt werden kann.

Das Endgerät weist als Schnittstellen neben dem Bildschirm als optisches Ausgabemodul bzw. Ausgabegerät in möglicher ergänzender Ausgestaltung mindestens ein akustisches Ausgabemodul bzw. Ausgabegerät, bspw. einen Lautsprecher, und/oder mindestens ein haptisches Ausgabemodul bzw. Ausgabegerät sowie mindestens ein Eingabemodul, bspw. einen Lautsprecher, eine Tastatur und/oder einen berührungsempfindlichen Bildschirm (Touchscreen) auf. Dabei ist das optische und/oder akustische Ausgabemodul dazu ausgebildet, die Person, bspw. im Fall des Bildschirms schriftlich und/oder im Fall des Lautsprechers akustisch, zu fragen, ob sie für ein jeweils dargestelltes ausrichtungssensitives Sehzeichen bzw. bei einem jeweils dargestellten ausrichtungssensitiven Sehzeichen den mindestens einen Markierungsabstand zwischen den Markierungen für den jeweiligen Ausrichtungswinkel ω noch erkennt oder nicht. Mindestens ein Eingabemodul, bspw. ein Antwortfeld, das auf dem Bildschirm dargestellt wird, ist dazu ausgebildet, eine jeweilige Antwort, die von der Person durch manuelle Betätigung des mindestens einen Eingabemoduls eingegeben wird, zu registrieren. Falls als das mindestens Eingabemodul ein Mikrofon des Endgeräts verwendet wird, wird von diesem eine mündliche Antwort der Person, üblicherweise auf Grundlage von Spracherkennung (Speech-Recognition) registriert. Hierbei ist es in Ausgestaltung auch möglich, dass ein Erkennen des mindestens einen Markierungsabstands durch die Person mit einer Abfrage des Ausrichtungswinkels ω des Sehzeichens kombiniert wird, wobei die Person den jeweiligen Ausrichtungswinkel ω in das Endgerät eingibt.

In weiterer Ausgestaltung ist die Recheneinheit des Endgeräts dazu ausgebildet, eine Software, bspw. eine Anwendung bzw. Applikation (App) zur Durchführung des vorgestellten Verfahrens auszuführen, wobei diese Software in einem Speicher des Endgeräts gespeichert und/oder, bspw. dem Endgerät, von einem Server bereitstellbar bzw. bereitzustellen ist.

Die erfindungsgemäße Applikation ist für ein mobiles Endgerät zum Ermitteln mindestens einer üblicherweise optometrischen Eigenschaft von mindestens einem Auge einer Person ausgebildet, wobei das Endgerät einen Bildschirm aufweist. Die Applikation ist und/oder wird auf dem mobilen Endgerät implementiert, bspw. in einem Speicher des Endgeräts gespeichert, wobei sie auf dem mobilen Endgerät läuft und dazu ausgebildet ist, zu veranlassen, auf dem Bildschirm ausrichtungssensitive Sehzeichen darzustellen, wobei jedes ausrichtungssensitive Sehzeichen eine geometrische Eigenschaft aufweist, die von einem Ausrichtungswinkel ω und mindestens einem Markierungsabstand zwischen mindestens jeweils zwei Markierungen des ausrichtungssensitiven Sehzeichens abhängig ist. Die Applikation ist dazu ausgebildet, für mindestens drei unterschiedliche Ausrichtungswinkel ω jeweils eine Schar von ausrichtungssensitiven Sehzeichen zu ermitteln, wobei bspw. mindestens zwei Punkte als Markierungen der ausrichtungssensitiven Sehzeichen jeweils einer Schar für einen Ausrichtungswinkel ω von Sehzeichen zu Sehzeichen unterschiedliche Abstände aufweisen, wobei die Applikation dazu ausgebildet ist, auf dem Bildschirm nacheinander für jeden Ausrichtungswinkel ω die ausrichtungssensitiven Sehzeichen darzustellen und dabei für einen jeweiligen Ausrichtungswinkel ω mit einem ausrichtungssensitiven Sehzeichen zu beginnen, bei dem der mindestens eine und/oder jeweilige Markierungsabstand zwischen den Markierungen einen üblicherweise definierbaren festen Wert hat, und danach sukzessiv weitere ausrichtungssensitive Sehzeichen mit jeweils anderem Punktabstand zwischen den bspw. als Punkte ausgebildeten Markierungen darzustellen, wobei der mindestens eine Markierungsabstand für die sukzessiv dargestellten Sehzeichen zu verändern bzw. veränderbar ist. Die Applikation ist zudem dazu ausgebildet, durch Abfragen der Person zu überprüfen, welcher minimale Markierungsabstand zwischen den Markierungen eines jeweils dargestellten Sehzeichens für den jeweiligen Ausrichtungswinkel $\omega$ für die Person noch erkennbar ist oder nicht und eine jeweilige Antwort der Person zu verarbeiten, wobei die Applikation dazu ausgebildet ist, für jeden Ausrichtungswinkel $\omega$ den minimalen Markierungsabstand als Grenzabstand g zu ermitteln.

Ein jeweils zu ermittelnder Grenzabstand ist derjenige Markierungsabstand zwischen Markierungen der Schar an Sehzeichen, die von dem mindestens einen Auge gerade noch aufgelöst werden können. Im Fall eines Gitters als mögliches Sehzeichen ist dies der Markierungsabstand, bspw. Linienabstand, zwischen zwei parallelen Gitterlinien des Gitters, die gerade noch auseinander haltbar sind. Bei einem Landoltring ist vorgesehen, dass dieser eine ausrichtungswinkelabhängige Ausnehmung bzw. Lücke als geometrische Eigenschaft aufweist, die durch zwei Punkte als Markierungen am Ende des Landoltrings begrenzt ist, die voneinander den jeweils eingestellten Messabstand aufweisen, wobei zwischen den Markierungen der minimale Markierungsabstand und somit eine minimale Breite der Ausnehmung bzw. Lücke in dem Landoltring ermittelt wird, die von dem mindestens einen Auge gerade noch erkannt werden kann.

Mit dem Verfahren und dem System ist es möglich, die Sehstärke und/oder Sehschärfe als optometrische Eigenschaft des mindestens einen Auges der Person durch die bspw. mobile Applikation bzw. Anwendung zu bestimmen, wobei die Person die optometrische Eigenschaft, bspw. Sehstärke und/oder Sehschärfe, selbst messen kann. Dazu ist in Ausgestaltung vorgesehen, das Sehvermögen als optometrische Eigenschaft des mindestens einen Auges bspw. mit Hilfe von Landoltringen zu bestimmen und anhand von für mindestens drei Ausrichtungswinkel $\omega$ ermittelten entsprechend drei Werten von Grenzabständen bspw. die Ellipse als charakteristische Funktion ausgehend von einer bekannten Ellipsengleichung zu fitten. Das sphärische Sehen ist dabei durch einen von der Ellipse umfassten Kreis mit umfasst, der einer Ellipse entspricht, bei der die kleine und große Halbachse a, b gleich sind und einem Radius dieses Kreises entsprechen. Mit einer Ellipse, bei der die kleine und große Halbachse a, b verschieden sind, ist es aufgrund eines Unterschieds der Halbachsen a, b und/oder eines Verhältnisses der Halbachsen a, b relativ zueinander möglich, Astigmatismus zu berücksichtigen. Mit der Ellipse ist es möglich, ein Sehvermögen und/oder einen Sehfehler über die auf dem Bildschirm dargestellten ausrichtungswinkelabhängigen Sehzeichen zu ermitteln. Unter Berücksichtigung der mindestens einen optometrischen Eigenschaft ist es möglich für die Person eine Sehhilfe, bspw. eine Brille oder Kontaktlinsen, herzustellen.

Mit dem Verfahren ist es auch möglich, einen Refraktionswert als optometrische Eigenschaft des mindestens einen Auges subjektiv zu bestimmen. Die mit dem bspw. digitalen Verfahren vorgestellte Ermittlung der optometrischen Eigenschaft des mindestens einen Auges ist auch für eine Heimnutzung geeignet, da keine zusätzlichen messtechnischen Gerätschaften, wie bspw. Linsen, erforderlich sind. Eine Messung der Sehschärfe (Visus) ist mit den auf dem Bildschirm bzw. Display angezeigten und/oder präsentierten Sehzeichen als Muster möglich. Anhand eines Feedbacks der Person wird auf die Sehschärfe geschlossen. Durch die Verwendung eines Sprachassistenten oder einer anderen Software zur Auswertung des Feedbacks kann das Verfahren und somit ein Sehtest von der Person selbst durchgeführt werden. Für die Refraktionsbestimmung wird von diesem Sehschärfewert auf die Refraktion, bestehend aus den Dioptrienwerten Sphäre, Zylinder und Achse, geschlossen. Falls der Sehfehler symmetrisch, also rein sphärisch ist, wird dieser nur durch den Wert, d. h. Dioptrienwert, der Sphäre beschrieben. Es ist auch möglich, aus einem singulären Wert der Sehschärfe auf einen vollständigen Satz aus Sphäre, Zylinder und Achse zu schließen, wie er für einen astigmatischen, also asymmetrischen Sehfehler vorliegt, wobei bei dem Verfahren über die ausrichtungssensitiven Sehzeichen auch die Direktionalität des asymmetrischen bzw. astigmatischen Sehfehlers berücksichtigt wird, die weitere Informationen liefert. Diese ausrichtungssensitiven Informationen werden zum Übertragen von Messungen der Sehschärfe zu Refraktionswerten verwendet. Eine jeweilige Messung der Sehstärke anhand eines ausrichtungssensitiven Sehzeichens beruht darauf, das Auflösungsvermögen des Auges zu ermitteln. Dieses Auflösungsvermögen wird durch den minimalen Markierungsabstand zwischen mindestens zwei individuellen Markierungen des Sehzeichens als optischen Stimuli definiert. Der kleinste Markierungsabstand, der von dem untersuchten Auge noch identifiziert werden kann, bestimmt die Sehschärfe als optometrische Eigenschaft. Die für diese Messungen verwendeten Sehzeichen bzw. Optotypen umfassen ein kritisches Detail, dessen Wert dem zu ermittelnden ausrichtungswinkelabhängigen Markierungsabstand entspricht. Mit dem Verfahren wird bspw. mit einer Visus-Messung ein experimenteller Zugang zum Auflösungsvermögen des Auges bereitgestellt. Weiterhin wird das Auflösungsvermögen des Auges mit der Punktspreizfunktion (Point Spread Function, PSF) mathematisch berechnet. Die PSF ist eine charakteristische Funktion für optische Systeme und somit auch für ein Auge. Sie entspricht einer Impulsantwortfunktion eines optischen Systems und somit des Auges, wobei mit dem Bildschirm als Lichtquelle auf der Retina ein ausgedehntes Bild des jeweils dargestellten Sehzeichens erzeugt wird. Hierbei ist die PSF in der Regel von Aberrationen, also der Sphäre, dem Zylinder und der Achse als Sehfehler abhängig (Watson, Andrew. (2015). Computing human optical point spread functions. Journal of vision. 15. 10.1167/15.2.26). Zusätzlich können noch Prozessparameter wie der Messabstand zwischen Bildschirm und Auge und/oder die Pupillengröße berücksichtigt werden. Aus der PSF wird die Modulationstransferfunktion MTF abgeleitet, welche die Auflösungsgrenze des Auges direkt beschreibt.

Gleichzeitig beschreibt die MTF auch den Kontrastübertrag aus einer Objektebene, also dem Bildschirm, auf dem die Sehzeichen dargestellt werden, in eine Bildebene, also der Netzhaut bzw. Retina. Daher beschreiben die Nullstellen der MTF die Auflösungsgrenze des Auges, da die üblicherweise zwei als Punkte ausgebildeten Markierungen einer bestimmten Grenzfrequenz, und somit eines bestimmten Markierungsabstands, d.h., des Grenzabstands, im Bild des Sehzeichens keinen Kontrast liefern. Da in Sehschärfe-Messungen die Grenzfrequenz $f_g$ ermittelt wird, kann hierdurch die Direktionalität des Sehfehlers bzw. der MTF ausgenutzt werden. Die Abhängigkeit der MTF von den Refraktionswerten ergibt sich nämlich durch einen Einfluss auf die Form der MTF. Während die MTF für rein sphärische Sehfehler kreisförmig ist, ergibt sich aus einem astigmatischen Sehfehler eine elliptische MTF. Die charakteristischen Werte der Halbachsen a, b und der Rotation $\Phi$ der MTF, bspw. der Ellipse, sind direkt proportional zur Sphäre gemäß einer ersten der beiden Halbachsen a, b, also der kleinen oder großen Halbachse a, b, proportional zur Größe (Sphäre + Zylinder) gemäß der anderen der beiden Halbachsen a, b und proportional zur Rotation $\Phi$ und somit dem Rotationswinkel $\phi$ der Ellipse, wobei die Ellipse bei der Rotation $\Phi$ um dem Rotationswinkel $\phi$, üblicherweise relativ zu der Abszisse des Koordinatensystems, rotiert bzw. gedreht wird.

Es versteht sich, dass die voranstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung ist anhand von Ausführungsformen in der Zeichnung schematisch dargestellt und wird unter Bezugnahme auf die Zeichnung schematisch und ausführlich beschrieben.

Figur 1 zeigt in schematischer Darstellung eine Ausführungsform des erfindungsgemäßen Systems bei Durchführung einer Ausführungsform des erfindungsgemäßen Verfahrens.

Figuren 2a, 2b, 2c, 2d, 2e, 2f, 2g zeigen Beispiele für einen Kreis und Ellipsen, die bei der Ausführungsform des erfindungsgemäßen Verfahrens berücksichtigt werden.

Figur 3 zeigt ein Flussdiagramm zu der Ausführungsform des erfindungsgemäßen Verfahrens.

Figur 4 zeigt eine weitere Ellipse, die bei der Ausführungsform des erfindungsgemäßen Verfahrens verwendet wird.

Figur 5 zeigt in schematischer Darstellung noch eine weitere Ellipse, die bei der Ausführungsform des erfindungsgemäßen Verfahrens verwendet wird.

Die Figuren werden zusammenhängend und übergreifend beschrieben, gleichen Komponenten sind dieselben Bezugszeichen zugeordnet.

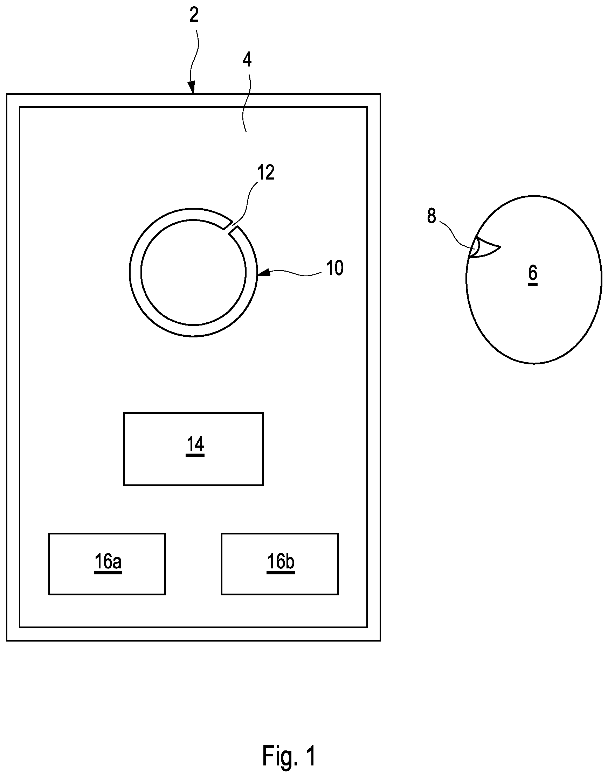

Die anhand von Figur 1 schematisch dargestellte Ausführungsform des erfindungsgemäßen Systems umfasst ein mobiles Endgerät 2, hier ein sogenanntes Smartphone, einer Person 6, das zur Datenverarbeitung und funkgestützten bzw. drahtlosen Kommunikation ausgebildet ist. Dabei umfasst dieses Endgerät 2 einen Bildschirm 4 bzw. ein Display. Zum Durchführen der Ausführungsform des erfindungsgemäßen Verfahrens ist vorab vorgesehen, dass in einen Speicher des Endgeräts 2 eine hierfür vorgesehene Anwendung bzw. Applikation (App) und somit Software bzw. ein entsprechendes Computerprogramm geladen wird. Diese Anwendung wird zum Durchführen des Verfahrens von einer Recheneinheit des Endgeräts 2 ausgeführt. Dabei wird für mindestens ein Auge 8 der Person 6 ein Sehtest durchgeführt, wobei eine optometrische Eigenschaft des Auges 8 ermittelt wird. Hierzu ist vorgesehen, dass der Bildschirm 4 des Endgeräts 2 in einem definierten Messabstand von dem Auge 8 der Person 6 entfernt angeordnet wird.

Bei dem Verfahren werden auf dem Bildschirm 4 Landoltringe 10 als ausrichtungssensitive Sehzeichen dargestellt. Dabei weist jeder Landoltring 10 eine Lücke 12 bzw. Öffnung auf, die eine von einem Ausrichtungswinkel $\omega$ abhängige Eigenschaft des Landoltrings 10 als Sehzeichen definiert. Diese Lücke 12 ist zwischen zwei Punkten als Markierungen des Landoltrings 10 angeordnet, wobei diese beiden Punkte zueinander einen Markierungsabstand aufweisen, der hier zugleich einer Breite der Lücke 12 des Landoltrings 10 entspricht. Dabei wird für mindestens drei unterschiedliche Ausrichtungswinkel $\omega$ jeweils eine Schar von ausrichtungssensitiven Landoltringen 10 als Sehzeichen ermittelt, wobei die zwei Punkte des ausrichtungssensitiven Landoltrings 10 jeweils einer Schar für einen Ausrichtungswinkel $\omega$ unterschiedliche Markierungsabstände aufweisen, wobei auf dem Bildschirm 4 nacheinander für jeden Ausrichtungswinkel $\omega$ die ausrichtungssensitiven Landoltringe 10 sukzessiv dargestellt werden, wobei der Markierungsabstand zwischen den beiden Punkten und somit die Breite der Lücke 12 variiert bzw. verändert wird.

Weiterhin wird für jeden jeweils dargestellten Landoltring 10 auf dem hier berührungsempfindlichen Bildschirm 4 bzw. Touchscreen ein Fragefeld 14 als Ausgabemodul eingeblendet, mit dem die Person 6 gefragt wird, ob sie den Markierungsabstand zwischen den beiden Punkten für den jeweiligen Ausrichtungswinkel $\omega$ des jeweils dargestellten Landoltrings 10 noch erkennen kann oder nicht. Zum Beantworten einer derartigen Frage ist hier vorgesehen, dass die Person 6 eines der beiden Antwortfelder 16a, 16b, die als Eingabemodule ebenfalls auf dem Bildschirm 4 dargestellt werden, manuell bedient. Falls die Person 6 und somit ihr Auge 8 den Markierungsabstand noch erkennen kann, ist bei der hier vorgestellten Ausführungsform des Verfahrens vorgesehen, dass sie ein erstes Antwortfeld 16a drückt und die Frage bejaht. Falls sie den Markierungsabstand nicht mehr erkennen kann, ist vorgesehen, dass sie ein zweites Antwortfeld 16b drückt und die Frage verneint.

Eine Anzeige bzw. Darstellung von veränderten Landoltringen 10 und eine Abfrage hinsichtlich einer Erkennbarkeit der Markierungsabstände wird so oft wiederholt, bis für jeden Ausrichtungswinkel $\omega$ der für die Person 6 bzw. ihr Auge 8 minimale noch erkennbare Markierungsabstand ermittelt wird, der als Grenzabstand g vorgesehen wird. Weiterhin wird aus einer Mehrzahl von ausrichtungssensitiven Grenzabständen g eine charakteristische Funktion des Auges 8 abgeleitet.

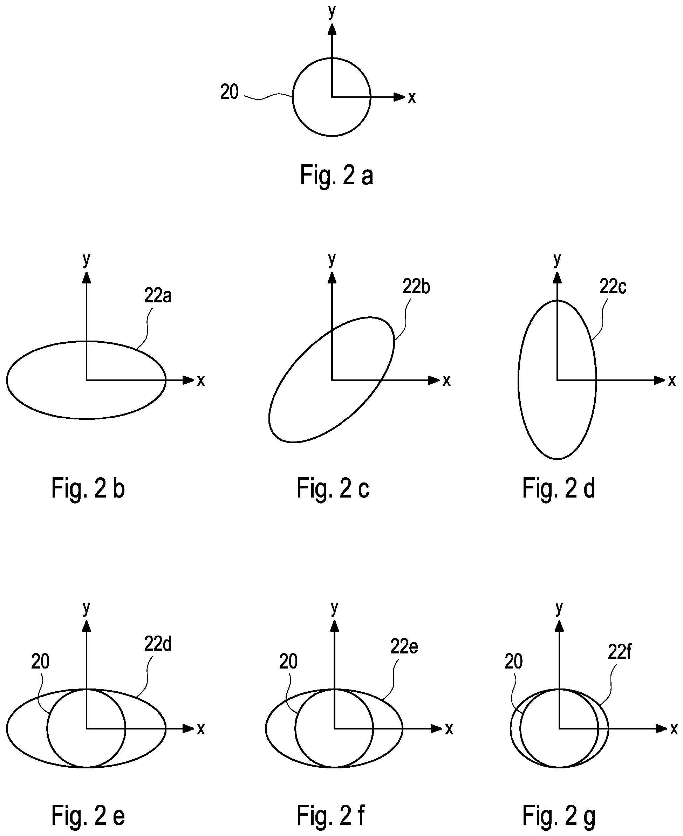

Bei der Ausführungsform des Verfahrens werden für die charakteristische Funktion rotierte Ellipsen 22a, 22b, 22c, 22d, 22e, 22f verwendet, die in den Figuren 2a, 2c, 2d, 2e, 2f, 2g jeweils in einem Koordinatensystem dargestellt sind, wobei dieses Koordinatensystem als Abszisse eine x-Achse und als Ordinate eine y-Achse aufweist. Dabei zeigen die Figuren 2a, 2e, 2f, 2g einen Kreis 20 als eine mögliche Ausführungsform einer Ellipse in dem Koordinatensystem, wobei der Kreis 20 in Figur 2a einer sphärischen bzw. kreisförmigen Ellipse entspricht. Hierbei ist weiterhin vorgesehen, dass der Kreis 20 bzw. eine jeweilige Ellipse 22a, 22b, 22c, 22d, 22e, 22f, die die charakteristische Funktion beschreiben, Nullstellen einer Modulationsübertragungsfunktion MTF in dem hier virtuellen Koordinatensystem beschreiben bzw. darstellen. Jede Ellipse 22a, 22b, 22c, 22d, 22e, 22f weist hier eine große Halbachse b und eine kleine Halbachse a auf. Bei dem Kreis 20 als Spezialfall einer rein sphärischen Ellipse sind die beiden Halbachsen a, b gleich groß und entsprechen somit einem Radius des Kreises 20.

Bei der ersten Ellipse 22a ist die große Halbachse b unter einem Rotationswinkel $\phi$ der Ellipse von 0° relativ zu der Abszisse orientiert. Bei der Ellipse 22b ist die große Halbachse unter einem Rotationswinkel $\phi$ der Ellipse von 45° relativ zu der Abszisse orientiert. Bei der Ellipse 22c ist die große Halbachse unter einem Rotationswinkel $\phi$ der Ellipse von 90° zu der Abszisse und somit parallel zu der Ordinate orientiert. In den Figuren 2e, 2f und 2g ist neben jeweils einer Ellipse 22d, 22e, 22f noch der Kreis 20 und somit eine entsprechende Sphäre dargestellt, wobei bei diesen drei Ellipsen 22d, 22e, 22f die große Halbachse unter einem Rotationswinkel $\phi$ der Ellipse von 0° relativ zu der Abszisse und somit parallel zu der Abszisse orientiert ist. Hierbei ist vorgesehen, dass der Kreis 20 einen Dioptrienwert von 1 andeutet. Die große Halbachse der Ellipse 2e deutet hier einen Dioptrienwert von 2 an. Die große Halbachse der Ellipse 22e deutet einen Dioptrienwert von 1,75 an. Bei der Ellipse 22f deutet die große Halbachse einen Dioptrienwert von 1,25 an. Dabei entspricht ein jeweiliger Dioptrienwert x einem Verhältnis bzw. einer Relation der großen Halbachse einer jeweiligen Ellipse 22d, 22e, 22f zu ihrer kleinen Halbachse und somit zu dem Radius des Kreises 20. Eine Rotation $\Phi$ einer jeweiligen Ellipse 22a, 22b, 22c, 22d, 22e, 22f als astigmatische MTF ergibt sich bei der hier gezeigten Ausführungsform aus dem Rotationswinkel $\phi$ der Ellipse 22a, 22b, 22c, 22d, 22e, 22f zwischen der Abszisse des Koordinatensystems und der großen Halbachse der Ellipse 22a, 22b, 22c, 22d, 22e, 22f. Somit zeigen die Figuren 2a, 2b, 2c, 2d, 2e, 2f, 2g eine Abhängigkeit einer Form einer Ellipse 22a, 22b, 22c, 22d als MTF von einem jeweiligen Refraktionswert als optometrische Eigenschaft des Auges 8, wobei die Form der Ellipse 22a, 22b, 22c, 22d, 22e, 22f durch ihre Halbachsen und den Rotationswinkel $\phi$ der Ellipse 22a, 22b, 22c, 22d, 22e, 22f definiert ist.

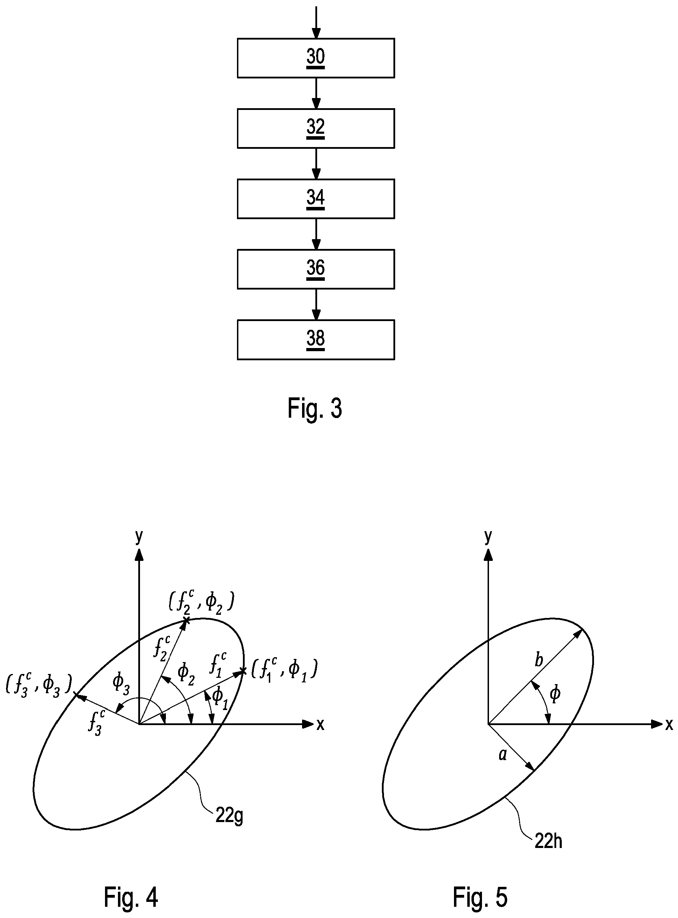

Das in Figur 3 dargestellte Flussdiagramm zeigt mehrere Schritte 30, 32, 34, 36, die bei der Ausführungsform des erfindungsgemäßen Verfahrens nacheinander durchgeführt werden. Bei der Ausführungsform des Verfahrens wird in Ausgestaltung in einem ersten Schritt 30 eine direktionale Messung einer Sehschärfe als optometrische Eigenschaft des Auges 8 durchgeführt. Dabei wird der Person 6 auf dem Bildschirm 4 zunächst ein erster Landoltring 10 dargestellt, bei dem die Lücke 12 unter einem Ausrichtungswinkel $\omega_1$ dargestellt wird. Dabei wird die Person 6 gefragt, ob sie die Lücke 12 optisch trennen bzw. auflösen kann oder nicht.

In einem zweiten Schritt 32 werden mindestens zwei zusätzliche direktionale Messungen für die Sehschärfe durchgeführt, wobei die Lücke 12 eines jeweiligen Landoltrings 10 unter einem Ausrichtungswinkel $\omega_i$ orientiert ist. Bei beiden Schritten 30, 32 werden für einen jeweiligen Ausrichtungswinkel $\omega_1$, $\omega_i$ Markierungsabstände zwischen Punkten eines jeweiligen Landoltrings 10, die dessen Lücke 12 begrenzen, variiert, bis für jeden Ausrichtungswinkel $\omega_1$, $\omega_i$ ein minimaler ausrichtungswinkelabhängiger Markierungsabstand ermittelt wird, der weiterhin als Grenzabstand bezeichnet wird, wobei aus einem Kehrwert des Grenzabstands eine jeweils ausrichtungswinkelabhängige Grenzfrequenz $f_g$ ermittelt wird.

In einem dritten Schritt 34 wird aus allen Grenzabständen g bzw. Grenzfrequenzen $f_g$ als Messpunkte eine rotierte Ellipse 22a, 22b, 22c, 22d, 22e, 22f ermittelt, für die ein mathematischer Fit und somit eine mathematische Anpassung berechnet wird, wobei für eine jeweilige Ellipse 22a, 22b, 22c, 22d, 22e, 22f die große Halbachse und die kleine Halbachse ermittelt wird.

In einem vierten Schritt wird der Refraktionswert als optometrische Eigenschaft des Auges 8 aus den charakteristischen Größen der Ellipse 22a, 22b, 22c, 22d, 22e, 22f, d. h. aus den beiden Halbachsen und dem Rotationswinkel $\phi$ der Ellipse 22a, 22b, 22c, 22d, 22e, 22f berechnet. Als Ergebnisse werden in einem fünften Schritt 36 die Sphäre, der Zylinder und die Achse ermittelt.

In dem Koordinatensystem aus Figur 4 ist die weitere Ellipse 22g dargestellt, für die hier drei Paare aus jeweils einer Grenzfrequenz $f_i^c$ und einem Rotationswinkel $\phi_i$ als Messwerte angegeben sind, wobei hier i 1, 2 oder 3 ist. Im Detail sind das für ein erstes Paar Messwerte eine erste Grenzfrequenz $f_1^c$ und ein erster Rotationswinkel $\phi_1$, für ein zweites Paar Messwerte eine zweite Grenzfrequenz $f_2^c$ und ein zweiter Rotationswinkel $\phi_2$ und für ein drittes Paar Messwerte eine dritte Grenzfrequenz $f_3^c$ und ein dritter Rotationswinkel $\phi_3$.

Durch Verwendung von ausrichtungssensitiven Landoltringen 10 als Optotypen bzw. Sehzeichen werden bei dem Verfahren ausrichtungsabhängige Grenzfrequenzen $f_i^c$ der Ellipse 22g als MTF bestimmt. Dabei wird aus einem Satz von mindestens drei Paaren an Messwerten $f_i^c$ und $\phi_i$ der Fit bzw. die Anpassung der Ellipse 22g ermittelt bzw. erstellt. Hierzu werden bspw. analytische Algorithmen, wie bspw. eine Least-Square-Methode bzw. Methode der kleinsten Quadrate, und/oder ein neuronales Netzwerk verwendet.In Ausgestaltung des Verfahrens ist vorgesehen, dass der analytische Algorithmus, der dem Endgerät 2 über die Anwendung (App) bereitgestellt wird, von diesem durchgeführt wird. Ferner ist es möglich, dass das Endgerät 2 einem neuronalen Netzwerk Daten austauscht, die von dem neuronalen Netzwerk verarbeitet werden, wobei das neuronale Netzwerk auf einer externen Recheneinheit, bspw. auf einem Server in einer Cloud implementiert und ausgeführt wird.

Darauf basierend wird eine weitere Ellipse 22h ermittelt, die in dem Koordinatensystem aus Figur 5 dargestellt wird. Für diese Ellipse 22h werden als charakteristische Ellipsengrößen deren kleine Halbachse a, deren große Halbachse b und deren Rotationswinkel $\phi$, der in Figur 5 durch die Rotation $\Phi$ repräsentiert ist, ermittelt, aus denen dann Refraktionswerte wie die Sphäre, der Zylinder und die Achse bestimmt werden. Hierbei ist es optional denkbar, dass bei einer derartigen Ermittlung auch andere zuvor ermittelte Prozessparameter, wie der Messabstand zwischen dem Auge 8 und dem auf dem Bildschirm 4 jeweils dargestellten Landoltring 10 und/oder die Pupillengröße des Auges 8 berücksichtigt werden.

Dabei werden nachfolgende Zusammenhänge berücksichtigt:

$$\text{kleine Halbachse a} = \varepsilon * \text{Sphäre}$$

$$\text{große Halbachse b} = \varepsilon * (\text{Sphäre} + \text{Zylinder})$$

$$b - a = \varepsilon * \text{Zylinder}$$

Hierbei ist $\varepsilon$ ein Proportionalitätsfaktor.

BEZUGSZEICHEN:

| | |
|---|---|
| 2 | Endgerät |
| 4 | Bildschirm |
| 6 | Person |
| 8 | Auge |
| 10 | Landoltring |
| 12 | Lücke |
| 14 | Fragefeld |
| 16a, 16b | Antwortfeld |
| 20 | Kreis |
| 22a, 22b 22c, 22d 22e, 22f 22g, 22h | Ellipse |
| 30, 32, 34, 36, 38 | Schritt |

## Patentansprüche

1.   Verfahren zum Ermitteln mindestens einer optometrischen Eigenschaft von mindestens einem Auge (8) einer Person (6) mit einem Endgerät (2), wobei auf einem Bildschirm (4) des Endgeräts (2) ausrichtungssensitive Sehzeichen dargestellt werden, wobei jedes ausrichtungssensitive Sehzeichen zwei Punkte als Markierungen aufweist, wobei eine geometrische Eigenschaft des Sehzeichens von einem Ausrichtungswinkel der zwei Markierungen und von einem Markierungsabstand zwischen den zwei Markierungen abhängig bzw. gebildet ist, wobei von dem Endgerät (2) für mindestens drei unterschiedliche Ausrichtungswinkel jeweils eine Schar von ausrichtungssensitiven Sehzeichen ermittelt wird, wobei die Markierungen der ausrichtungssensitiven Sehzeichen jeweils einer Schar für einen Ausrichtungswinkel von Sehzeichen zu Sehzeichen unterschiedliche Markierungsabstände aufweisen, wobei auf dem Bildschirm (4) nacheinander für jeden Ausrichtungswinkel die ausrichtungssensitiven Sehzeichen dargestellt werden, wobei für einen jeweiligen Ausrichtungswinkel mit einem ausrichtungssensitiven Sehzeichen begonnen wird, bei dem der jeweilige Markierungsabstand zwischen den Markierungen einen definierten Wert aufweist, wobei danach sukzessiv weitere ausrichtungssensitive Sehzeichen mit jeweils einem anderen Markierungsabstand zwischen Markierungen dargestellt werden, wobei bestimmt wird, welcher minimale Markierungsabstand zwischen den Markierungen für den jeweiligen Ausrichtungswinkel für die Person (6) noch erkennbar ist, wobei das Endgerät (2) mindestens ein Ausgabemodul und mindestens ein Eingabemodul aufweist, wobei das mindestens eine Ausgabemodul dazu ausgebildet ist, die Person (6) zu fragen, ob sie für ein jeweils dargestelltes ausrichtungssensitives Sehzeichen den Markierungsabstand zwischen den Markierungen für den jeweiligen Ausrichtungswinkel noch erkennt oder nicht, wobei das mindestens eine Eingabemodul dazu ausgebildet ist, eine jeweilige Antwort, die von der Person (6) durch Betätigung des mindestens einen Eingabemoduls eingegeben wird, zu registrieren, wobei für jeden Ausrichtungswinkel der minimale Markierungsabstand als Grenzabstand g ermittelt wird, wobei von dem Endgerät (2) aus einer Mehrzahl von Grenzabständen g eine charakteristische Funktion für das mindestens eine Auge (8) abgeleitet wird, wobei die charakteristische Funktion eine Nullstellenfunktion einer Modulationsübertragungsfunktion MTF in einem virtuellen Koordinatensystem beschreibt, wobei die Nullstellen der Modulationsübertragungsfunktion MTF eine Auflösungsgrenze des Auges (8) beschreiben, wobei die als Punkte ausgebildeten Markierungen des Sehzeichens einer Grenzfrequenz und somit des Grenzabstands g im Bild des Sehzeichens keinen Kontrast liefern, wobei die Grenzfrequenz jeweils aus dem Kehrwert des entsprechenden ausrichtungswinkelabhängigen Grenzabstands berechnet wird.

2. Verfahren nach Anspruch 1, bei dem für die charakteristische Funktion eine rotierte Ellipse (22a, 22b, 22c, 22d, 22e, 22f, 22g, 22h) mit einem Fit angepasst wird, wobei aus mindestens drei ausrichtungswinkelabhängigen Grenzabständen g eine große Halbachse, eine kleine Halbachse und ein Rotationswinkel der Ellipse (22a, 22b, 22c, 22d, 22e, 22f, 22g, 22h) als Ellipsenparameter ermittelt werden.

3. Verfahren nach Anspruch 2, bei dem aus den Ellipsenparametern der rotierten Ellipse (22a, 22b, 22c, 22d, 22e, 22f, 22g, 22h) als Geometrieparameter eine primäre Sphäre, ein primärer Zylinder und eine primäre Achse ermittelt werden, wobei aus mindestens einem Geometrieparameter mindestens ein Dioptrienwert ermittelt wird, und/oder wobei mindestens ein Verhältnis aus zwei der Geometrieparameter relativ zueinander ermittelt wird.

4. Verfahren nach Anspruch 3, bei dem die primäre Sphäre, der primäre Zylinder und die primäre Achse als Geometrieparameter durch einen Einsatz einer künstlichen Intelligenz unter anderem aus den ausrichtungswinkelabhängigen Grenzabständen g ermittelt werden.

5. Verfahren nach Anspruch 4, bei dem durch Einbezug von mindestens einem weiteren Prozessparameter aus dem Verhältnis der primären Sphäre und des primären Zylinders eine sekundäre Sphäre und ein sekundärer Zylinder als Dioptrienwerte ermittelt werden.

6. Verfahren nach Anspruch 5, bei dem der mindestens eine weitere Prozessparameter ein Messabstand des mindestens einen Auges (8) der Person (6) zu dem Bildschirm (4) des Endgeräts (2) und/oder die Pupillengröße von dem mindestens einen Auge (8) der Person (6) ist.

7. Verfahren nach Anspruch 5 oder 6, bei dem der mindestens eine weitere Prozessparameter durch eine Sensorik des Endgeräts (2) erfasst wird.

8. Verfahren nach einem der voranstehenden Ansprüche, bei dem die Modulationsübertragungsfunktion MTF durch:

$$MTF(f) = |PSF(f)|^2 = \text{Bildkontrast } (f)/ \text{ Objektkontrast } (f)$$

gebildet wird, wobei PSF eine Punktspreizfunktion ist, und wobei als Ortsfrequenz f ein ausrichtungswinkelabhängiger Grenzabstand g verwendet wird.

9. Verfahren nach einem der voranstehenden Ansprüche, bei dem als ausrichtungssensitive Sehzeichen Landoltringe (10) verwendet werden, wobei jeder ausrichtungssensitive Landoltring (10) als ausrichtungswinkelabhängige Eigenschaft eine Lücke (12) aufweist, wobei jede Lücke (12) durch zwei Punkte als Markierungen begrenzt ist, die Enden des ausrichtungssensitiven Landoltrings (10) bilden, wobei von dem Endgerät (2) für jeden Ausrichtungswinkel eine Schar an ausrichtungssensitiven Landoltringen (10) dargestellt wird, wobei Enden der ausrichtungssensitiven Landoltringe (10) zueinander einen Punktabstand als Markierungsabstand aufweisen.

10. System zum Ermitteln mindestens einer optometrischen Eigenschaft von mindestens einem Auge (8) einer Person (6), wobei das System ein Endgerät (2) mit einem Bildschirm (4) und einer Recheneinheit aufweist, wobei das Endgerät (2) dazu ausgebildet ist, auf dem Bildschirm (4) ausrichtungssensitive Sehzeichen darzustellen, wobei jedes ausrichtungssensitive Sehzeichen zwei Punkte als Markierungen aufweist, wobei eine geometrische Eigenschaft des Sehzeichens von einem Ausrichtungswinkel der zwei Markierungen und von einem Markierungsabstand zwischen den zwei Markierungen abhängig bzw. gebildet ist, wobei das Endgerät (2) dazu ausgebildet ist, für mindestens drei unterschiedliche Ausrichtungswinkel jeweils eine Schar von ausrichtungssensitiven Sehzeichen zu ermitteln, wobei die Markierungen der ausrichtungssensitiven Sehzeichen jeweils einer Schar für einen Ausrichtungswinkel von Sehzeichen zu Sehzeichen unterschiedliche Markierungsabstände aufweisen, wobei das Endgerät (2) dazu ausgebildet ist, auf dem Bildschirm (4) nacheinander für jeden Ausrichtungswinkel die ausrichtungssensitiven Sehzeichen darzustellen und dabei für einen jeweiligen Ausrichtungswinkel mit einem ausrichtungssensitiven Sehzeichen zu beginnen, bei dem der jeweilige Markierungsabstand zwischen den Markierungen ein definierter Wert ist, und danach sukzessiv weitere ausrichtungssensitive Sehzeichen mit jeweils einem anderen Markierungsabstand zwischen den Markierungen darzustellen, wobei das Endgerät (2) dazu ausgebildet ist, zu überprüfen, welcher minimale Markierungsabstand zwischen den Markierungen für den jeweiligen Ausrichtungswinkel für die Person (6) noch erkennbar ist, wobei das Endgerät (2) mindestens ein Ausgabemodul und mindestens ein Eingabemodul aufweist, wobei das mindestens eine Ausgabemodul dazu ausgebildet ist, die Person (6) zu fragen, ob sie für ein jeweils dargestelltes ausrichtungssensitives Sehzeichen den Markierungsabstand zwischen den Markierungen für den jeweiligen Ausrichtungswinkel noch erkennt oder nicht, wobei das mindestens eine Eingabemodul dazu ausgebildet ist, eine jeweilige Antwort, die von der Person (6) durch Betätigung des mindestens einen Eingabemoduls eingegeben wird, zu registrieren, wobei das Endgerät (2) dazu ausgebildet ist, für jeden Ausrichtungswinkel den minimalen Markierungsabstand als Grenzabstand g zu ermitteln, wobei von dem Endgerät (2) aus einer Mehrzahl von Grenzabständen g eine charakteristische Funktion für das mindestens eine Auge (8) abgeleitet wird, wobei die charakteristische Funktion eine Nullstellenfunktion einer Modulationsübertragungsfunktion MTF in einem virtuellen Koordinatensystem beschreibt, wobei die Nullstellen der Modulationsübertragungsfunktion MTF eine Auflösungsgrenze des Auges (8) beschreiben, wobei die als Punkte ausgebildeten Markierungen des Sehzeichens einer Grenzfrequenz und somit des Grenzabstands g im Bild des Sehzeichens keinen Kontrast liefern, wobei die Grenzfrequenz jeweils aus dem Kehrwert des entsprechenden ausrichtungswinkelabhängigen Grenzabstands berechnet wird.

11. System nach Anspruch 10, bei dem die Recheneinheit dazu ausgebildet ist, aus mindestens drei Grenzabständen g für entsprechend mindestens drei unterschiedlichen Ausrichtungswinkel als Nullstellen einer Modulationsübertragungsfunktion MTF in einem virtuellen Koordinatensystem eine rotierte Ellipse (22a, 22b, 22c, 22d, 22e, 22f, 22g, 22h) mit einem Fit anzupassen und aus den mindestens drei ausrichtungswinkelabhängigen Grenzabständen g eine große Halbachse, eine kleine Halbachse und einen Rotationswinkel der Ellipse (22a, 22b, 22c, 22d, 22e, 22f, 22g, 22h) zu ermitteln.

12. System nach Anspruch 10 oder 11, bei dem das Endgerät (2) als mobiles Datenverarbeitungs- und Kommunikationsgerät ausgebildet ist.

13. System nach einem der Ansprüche 10 bis 12, bei dem die Recheneinheit dazu ausgebildet ist, eine Software zur Durchführung eines Verfahrens nach einem der Ansprüche 1 bis 9 auszuführen, wobei diese Software in einem Speicher des Endgeräts (2) gespeichert und/oder von einem Server bereitzustellen ist.

14. Applikation für ein Endgerät (2) zum Ermitteln mindestens einer optometrischen Eigenschaft von mindestens einem Auge (8) einer Person (6), wobei das Endgerät (2) einen Bildschirm (4) aufweist, wobei die Applikation auf dem Endgerät (2) implementiert ist, auf dem Endgerät (2) läuft und dazu ausgebildet ist, auf dem Bildschirm (4) ausrichtungssensitive Sehzeichen darzustellen, wobei jedes ausrichtungssensitive Sehzeichen zwei Punkte als Markierungen aufweist, wobei eine geometrische Eigenschaft des Sehzeichens von einem Ausrichtungswinkel der zwei Markierungen und von einem Markierungsabstand zwischen den zwei Markierungen abhängig bzw. gebildet ist,, wobei die Applikation dazu ausgebildet ist, für mindestens drei unterschiedliche Ausrichtungswinkel jeweils eine Schar von ausrichtungssensitiven Sehzeichen zu ermitteln, wobei die Markierungen der ausrichtungssensitiven Sehzeichen jeweils einer Schar für einen Ausrichtungswinkel von Sehzeichen zu Sehzeichen unterschiedliche Markierungsabstände aufweisen, wobei die Applikation dazu ausgebildet ist, auf dem Bildschirm (4) nacheinander für jeden Ausrichtungswinkel die ausrichtungssensitiven Sehzeichen darzustellen und dabei für einen jeweiligen Ausrichtungswinkel mit einem ausrichtungssensitiven Sehzeichen zu beginnen, bei dem der jeweilige Markierungsabstand zwischen den Markierungen einen definierten Wert aufweist, und danach sukzessiv weitere ausrichtungssensitive Sehzeichen mit jeweils einem anderen Markierungsabstand zwischen den Markierungen darzustellen, wobei die Applikation dazu ausgebildet ist, zu überprüfen, welcher minimale Markierungsabstand zwischen den Markierungen für den jeweiligen Ausrichtungswinkel für die Person (6) noch erkennbar ist, wobei das Endgerät (2) mindestens ein Ausgabemodul und mindestens ein Eingabemodul aufweist, wobei das mindestens eine Ausgabemodul dazu ausgebildet ist, die Person (6) zu fragen, ob sie für ein jeweils dargestelltes ausrichtungssensitives Sehzeichen den Markierungsabstand zwischen den Markierungen für den jeweiligen Ausrichtungswinkel noch erkennt oder nicht, wobei das mindestens eine Eingabemodul dazu ausgebildet ist, eine jeweilige Antwort, die von der Person (6) durch Betätigung des mindestens einen Eingabemoduls eingegeben wird, zu registrieren, wobei die Applikation dazu ausgebildet ist, für jeden Ausrichtungswinkel den minimalen Markierungsabstand als Grenzabstand g zu ermitteln, wobei von dem Endgerät (2) aus einer Mehrzahl von Grenzabständen g eine charakteristische Funktion für das mindestens eine Auge (8) abgeleitet wird, wobei die charakteristische Funktion eine Nullstellenfunktion einer Modulationsübertragungsfunktion MTF in einem virtuellen Koordinatensystem beschreibt, wobei die Nullstellen der Modulationsübertragungsfunktion MTF eine Auflösungsgrenze des Auges (8) beschreiben, wobei die als Punkte ausgebildeten Markierungen des Sehzeichens einer Grenzfrequenz und somit des Grenzabstands g im Bild des Sehzeichens keinen Kontrast liefern, wobei die Grenzfrequenz jeweils aus dem Kehrwert des entsprechenden ausrichtungswinkelabhängigen Grenzabstands berechnet wird.

## Claims

1. A method for determining at least one optometric property of at least one eye (8) of a person (6) with a terminal (2), wherein alignment-sensitive visual cues are displayed on a screen (4) of the terminal (2), wherein each alignment-sensitive visual cue has two points as markings, wherein a geometric property of the visual cue is dependent on or formed by an alignment angle of the two markings and a marking distance between the two markings, wherein respectively one set of alignment-sensitive visual cues is determined by the terminal (2) for at least three different orientation angles, wherein the markings of the alignment-sensitive visual cues of respectively one set have different marking distances for an alignment angle from visual cue to visual cue, wherein the alignment-sensitive visual cues are displayed successively on the screen (4) for each alignment angle, wherein an alignment-sensitive visual cue at which the respective marking distance between the markings has a defined value is used to start for a respective alignment angle, wherein further alignment-sensitive visual cues are then successively displayed with respectively one other marking distance between markings, wherein it is determined which minimum marking distance between the markings for the respective alignment angle is still recognizable for the person (6), wherein the terminal (2) has at least one output module and at least one input module, wherein the at least one output module is configured to ask the person (6) whether or not the person still recognizes the marking distance between the markings for the respective alignment angle for a respectively displayed alignment-sensitive visual cue, wherein the at least one input module is configured to register a respective response entered by the person (6) by actuating the at least one input module, wherein for each alignment angle the minimum marking distance is determined as a limit distance g, wherein a characteristic function for the at least one eye (8) is derived by the terminal (2) from a plurality of limit distances g, wherein the characteristic function describes a zero crossing function of a modulation transfer function MTF in a virtual coordinate system, wherein the zeros of the modulation transfer function MTF describe a resolution limit of the eye (8), wherein the markings, configured as points, of the visual cue of a limit frequency and thus of the limit distance g in the image of the visual cue provide no contrast, wherein the limit frequency is calculated from the inverse value of the corresponding alignment-angle dependent limit distance.

2. The method according to claim 1, wherein for the characteristic function a rotated ellipse (22a, 22b, 22c, 22d, 22e, 22f, 22g, 22h) is adjusted with a fit, wherein a major semi- axis, a minor semi- axis and a rotation angle of the ellipse (22a, 22b, 22c, 22d, 22e, 22f, 22g, 22h) are determined as ellipse parameters from at least three alignment-angle dependent limiting distances g.

3. The method according to claim 2, wherein from the ellipse parameters of the rotated ellipse (22a, 22b, 22c, 22d, 22e, 22f, 22g, 22h) a primary sphere, a primary cylinder and a primary axis are determined as geometry parameters, wherein from at least one geometry parameter at least one diopter value is determined, and/or wherein at least one ratio of two of the geometry parameters relative to each other is determined.

4. The method according to claim 3, wherein the primary sphere, the primary cylinder and the primary axis are determined as geometry parameters from, inter alia, the alignment-angle dependent limiting distances g by a use of artificial intelligence.

5. The method according to claim 4, wherein a secondary sphere and a secondary cylinder are determined as diopter values from the ratio of the primary sphere and the primary cylinder by including at least one further process parameter.

6. The method according to claim 5, wherein the at least one further process parameter is a measuring distance of the at least one eye (8) of the person (6) to the screen (4) of the terminal (2) and/or the pupil size of the at least one eye (8) of the person (6).

7. The method according to claim 5 or 6, wherein the at least one further process parameter is acquired by a sensor system of the terminal (2).

8. The method according to one of the preceding claims, wherein the modulation transfer function MTF is formed by:

$$MTF(f) = |PSF(f)|^2 = \text{image contrast } (f) / \text{object contrast } (f)$$

wherein PSF is a point spread function, and wherein an alignment-angle dependent limiting distance g is used as the spatial frequency f.

9. The method according to one of the preceding claims, wherein Landolt rings (10) are used as alignment-sensitive visual cues, wherein each alignment-sensitive Landolt ring (10) has a gap (12) as an alignment-angle dependent property, wherein each gap (12) is limited by two points as markings, which form ends of the alignment-sensitive Landolt ring (10), wherein a set of alignment-sensitive Landolt rings (10) is displayed by the terminal (2) for each alignment angle, wherein ends of the alignment-sensitive Landolt rings (10) have a point distance from each other as marking distance.

10. A system for determining at least one optometric property of at least one eye (8) of a person (6), wherein the system has a terminal (2) with a screen (4) and a computing unit, wherein the terminal (2) is configured to display alignment-sensitive visual cues on the screen (4), wherein each alignment-sensitive visual cue has two points as markings, wherein a geometric property of the visual cue is dependent on or formed by an alignment angle of the two markings and a marking distance between the two markings, wherein the terminal (2) is configured to respectively determine one set of alignment-sensitive visual cues for at least three different orientation angles, wherein the markings of the alignment-sensitive visual cues of respectively one set have different marking distances for an alignment angle from visual cue to visual cue, wherein the terminal (2) is configured to display the alignment-sensitive visual cues successively on the screen (4) for each alignment angle and to start that action with an alignment-sensitive visual cue at which the respective marking distance between the markings is a defined value, and to then display further alignment-sensitive visual cues successively with respectively one other marking distance between the markings, wherein the terminal (2) is configured to check, which minimum marking distance between the markings for the respective alignment angle is still recognizable for the person (6), wherein the terminal (2) has at least one output module and at least one input module, wherein the at least one output module is configured to ask the person (6) whether or not the person still recognizes the marking distance between the markings for the respective alignment angle for a respectively displayed alignment-sensitive visual cue, wherein the at least one input module is configured to register a respective response entered by the person (6) by actuating the at least one input module, wherein the terminal (2) is configured to determine for each alignment angle the minimum marking distance as a limit distance g, wherein a characteristic function for the at least one eye (8) is derived by the terminal (2) from a plurality of limit distances g, wherein the characteristic function describes a zero crossing function of a modulation transfer function MTF in a virtual coordinate system, wherein the zeros of the modulation transfer function MTF describe a resolution limit of the eye (8), wherein the markings, configured as points, of the visual cue of a limit frequency and thus of the limit distance g in the image of the visual cue provide no contrast, wherein the limit frequency is calculated from the inverse value of the corresponding alignment-angle dependent limit distance.

11. The system according to claim 10, in which the computing unit is configured to adjust a rotated ellipse (22a, 22b, 22c, 22d, 22e, 22f, 22g, 22h) with a fit from at least three limiting distances g for correspondingly at least three different alignment angles as zeros of a modulation transfer function MTF in a virtual coordinate system and to determine a major semi- axis, a minor semi- axis and a rotation angle of the ellipse (22a, 22b, 22c, 22d, 22e, 22f, 22g, 22h) from the at least three alignment-angle dependent limiting distances g.

12. The system according to claim 10 or 11, wherein the terminal (2) is configured as a mobile data processing and communication device.

13. The system according to one of claims 10 to 12, wherein the computing unit is configured to execute a software for performing a method according to one of claims 1 to 9, wherein said software is stored in a memory of the terminal (2) and/or is to be provided by a server.

14. An application for a terminal (2) for determining at least one optometric property of at least one eye (8) of a person (6), wherein the terminal (2) has a screen (4), wherein the application is implemented on the terminal (2), runs on the terminal (2) and is configured to display alignment-sensitive visual cues on the screen (4), wherein each alignment-sensitive visual cue has two points as markings, wherein a geometric property of the visual cue is dependent on or formed by an alignment angle of the two markings and a marking distance between the two markings, wherein the application is configured to respectively determine one set of alignment-sensitive visual cues for at least three different orientation angles, wherein the markings of the alignment-sensitive visual cues of respectively one set have different marking distances for an alignment angle from visual cue to visual cue, wherein the application is configured to display the alignment-sensitive visual cues successively on the screen (4) for each alignment angle and to start that action with an alignment-sensitive visual cue at which the respective marking distance between the markings has a defined value, and to then display further alignment-sensitive visual cues successively with respectively one other marking distance between the markings, wherein the application is configured to check, which minimum marking distance between the markings for the respective alignment angle is still recognizable for the person (6), wherein the terminal (2) has at least one output module and at least one input module, wherein the at least one output module is configured to ask the person (6) whether or not the person still recognizes the marking distance between the markings for the respective alignment angle for a respectively displayed alignment-sensitive visual cue, wherein the at least one input module is configured to register a respective response entered by the person (6) by actuating the at least one input module, wherein the application is configured to determine for each alignment angle the minimum marking distance as a limit distance g, wherein a characteristic function for the at least one eye (8) is derived by the terminal (2) from a plurality of limit distances g, wherein the characteristic function describes a zero crossing function of a modulation transfer function MTF in a virtual coordinate system, wherein the zeros of the modulation transfer function MTF describe a resolution limit of the eye (8), wherein the markings, configured as points, of the visual cue of a limit frequency and thus of the limit distance g in the image of the visual cue provide no contrast, wherein the limit frequency is calculated from the inverse value of the corresponding alignment-angle dependent limit distance.

## Revendications

1. Procédé de détermination d'au moins une caractéristique optométrique d'au moins un œil (8) d'une personne (6) au moyen d'un terminal (2), des signes visuels sensibles à l'orientation étant affichés sur un écran (4) du terminal (2), chaque signe visuel sensible à l'orientation présentant deux points en tant que repères, une propriété géométrique du signe visuel étant dépendante d'un angle d'orientation des deux repères et d'une distance de repérage entre les deux repères ou formée par celui-ci, le terminal (2) déterminant pour au moins trois angles d'orientation différents à chaque fois un groupe de signes visuels sensibles à l'orientation, les repères des signes visuels sensibles à l'orientation de chaque groupe présentant des distances de repérage différentes d'un signe visuel à l'autre pour un angle d'orientation, les signes visuels sensibles à l'orientation étant affichés successivement à l'écran (4) pour chaque angle d'orientation, en commençant, pour chaque angle d'orientation, par un signe visuel sensible à l'orientation pour lequel la distance de repérage respective entre les repères présente une valeur définie, d'autres signes visuels sensibles à l'orientation étant ensuite successivement affichés avec à chaque fois une autre distance de repérage entre des repères, en déterminant quelle distance de repérage minimale entre les repères est encore reconnaissable par la personne (6) pour l'angle d'orientation respectif, le terminal (2) présentant au moins un module de sortie et au moins un module d'entrée, l'au moins un module de sortie étant conçu pour demander à la personne (6) si, pour un signe visuel sensible à l'orientation respectivement affiché, elle reconnaît encore ou non la distance de repérage entre les repères pour l'angle d'orientation respectif, l'au moins un module d'entrée étant conçu pour enregistrer une réponse respective qui est introduite par la personne (6) en actionnant l'au moins un module d'entrée, la distance de repérage minimale étant déterminée en tant que distance limite g pour chaque angle d'orientation, une fonction caractéristique pour l'au moins un œil (8) étant déduite par le terminal (2) à partir d'une pluralité de distances limites g, la fonction caractéristique décrivant une fonction de point zéro d'une fonction de transfert de modulation MTF dans un système de coordonnées virtuel, les points zéro de la fonction de transfert de modulation MTF décrivant une limite de résolution de l'œil (8), les repères, réalisés sous la forme de points, du signe visuel d'une fréquence limite et donc de la distance limite g dans l'image du signe visuel ne fournissant aucun contraste, la fréquence limite étant calculée à chaque fois à partir de l'inverse de la distance limite correspondante dépendante de l'angle d'orientation.

2. Procédé selon la revendication 1, dans lequel, pour la fonction caractéristique, une ellipse (22a, 22b, 22c, 22d, 22e, 22f, 22g, 22h) pivotée est personnalisée par un ajustement, un grand demi-axe, un petit demi-axe et un angle de rotation de l'ellipse (22a, 22b, 22c, 22d, 22e, 22f, 22g, 22h) étant déterminés en tant que paramètres d'ellipse à partir d'au moins trois distances limites g dépendantes de l'angle d'orientation.

3. Procédé selon la revendication 2, dans lequel une sphère primaire, un cylindre primaire et un axe primaire sont déterminés en tant que paramètres géométriques à partir des paramètres d'ellipse de l'ellipse (22a, 22b, 22c, 22d, 22e, 22f, 22g, 22h) pivotée, au moins une valeur dioptrique étant déterminée à partir d'au moins un paramètre géométrique et/ou au moins un rapport entre deux des paramètres géométriques l'un par rapport à l'autre étant déterminé .

4. Procédé selon la revendication 3, dans lequel la sphère primaire, le cylindre primaire et l'axe primaire sont déterminés en tant que paramètres géométriques par une utilisation d'une intelligence artificielle, notamment à partir des distances limites g dépendant de l'angle d'orientation.

5. Procédé selon la revendication 4, dans lequel une sphère secondaire et un cylindre secondaire sont déterminés en tant que valeurs dioptriques à partir du rapport de la sphère primaire et du cylindre primaire en intégrant au moins un autre paramètre de processus.

6. Procédé selon la revendication 5, dans lequel l'au moins un autre paramètre de processus est une distance de mesure de l'au moins un œil (8) de la personne (6) par rapport à l'écran (4) du terminal (2) et/ou la taille de la pupille de l'au moins un œil (8) de la personne (6).

7. Procédé selon la revendication 5 ou 6, dans lequel l'au moins un autre paramètre de processus est saisi par un système de détection du terminal (2).

8. Procédé selon l'une des revendications précédentes, dans lequel la fonction de transfert de modulation MTF est définie par :

$$MTF(f) = |PSF(f)|^2 = \text{contraste d'image (f) / contraste d'objet (f)}$$

où PSF est une fonction d'expansion des points et où une distance limite g dépendant de l'angle d'orientation est utilisée en tant que fréquence spatiale f.

9. Procédé selon l'une des revendications précédentes, dans lequel des anneaux de Landolt (10) sont utilisés en tant que repères sensibles à l'orientation, chaque anneau de Landolt (10) sensible à l'orientation présentant un espace (12) en tant que propriété dépendante de l'angle d'orientation, chaque espace (12) étant délimité par deux points en tant que repères qui forment les extrémités de l'anneau de Landolt (10) sensible à l'orientation, un groupe d'anneaux de Landolt (10) sensibles à l'orientation étant affiché par le terminal (2) pour chaque angle d'orientation, les extrémités des anneaux de Landolt (10) sensibles à l'orientation présentant les unes par rapport aux autres un espacement de points servant de distance de repérage.

10. Système de détermination d'au moins une caractéristique optométrique d'au moins un œil (8) d'une personne (6), le système présentant un terminal (2) doté d'un écran (4) et d'une unité de calcul, le terminal (2) étant conçu pour afficher des signes visuels sensibles à l'orientation à l'écran (4), chaque signe visuel sensible à l'orientation présentant deux points en tant que repères, une propriété géométrique du signe visuel étant dépendante d'un angle d'orientation des deux repères et d'une distance de repérage entre les deux repères ou formée par celui-ci, le terminal (2) étant conçu pour déterminer pour au moins trois angles d'orientation différents à chaque fois un groupe de signes visuels sensibles à l'orientation, les repères des signes visuels sensibles à l'orientation de chaque groupe présentant des distances de repérage différentes d'un signe visuel à l'autre pour un angle d'orientation, le terminal (2) étant conçu pour afficher successivement à l'écran (4) les signes visuels sensibles à l'orientation pour chaque angle d'orientation, en commençant, pour chaque angle d'orientation, par un signe visuel sensible à l'orientation pour lequel la distance de repérage respective entre les repères présente une valeur définie, et pour afficher ensuite successivement d'autres signes visuels sensibles à l'orientation avec à chaque fois une autre distance de repérage entre des repères, le terminal (2) étant conçu pour vérifier quelle distance de repérage minimale entre les repères est encore reconnaissable par la personne (6) pour l'angle d'orientation respectif, le terminal (2) présentant au moins un module de sortie et au moins un module d'entrée, l'au moins un module de sortie étant conçu pour demander à la personne (6) si, pour un signe visuel sensible à l'orientation respectivement affiché, elle reconnaît encore ou non la distance de repérage entre les repères pour l'angle d'orientation respectif, l'au moins un module d'entrée étant conçu pour enregistrer une réponse respective qui est introduite par la personne (6) en actionnant l'au moins un module d'entrée, le terminal (2) étant conçu pour déterminer la distance de repérage minimale en tant que distance limite g pour chaque angle d'orientation, une fonction caractéristique pour l'au moins un œil (8) étant déduite par le terminal (2) à partir d'une pluralité de distances limites g, la fonction caractéristique décrivant une fonction de point zéro d'une fonction de transfert de modulation MTF dans un système de coordonnées virtuel, les points zéro de la fonction de transfert de modulation MTF décrivant une limite de résolution de l'œil (8), les repères, réalisés sous la forme de points, du signe visuel d'une fréquence limite et donc de la distance limite g dans l'image du signe visuel ne fournissant aucun contraste, la fréquence limite étant calculée à chaque fois à partir de l'inverse de la distance limite correspondante dépendante de l'angle d'orientation.

11. Système selon la revendication 10, dans lequel l'unité de calcul est conçue pour personnaliser une ellipse (22a, 22b, 22c, 22d, 22e, 22f, 22g, 22h) pivotée à partir d'au moins trois distances limites g, pour au moins trois angles d'orientation différents correspondants en tant que points zéro d'une fonction de transfert de modulation MTF dans un système de coordonnées virtuel et pour déterminer un grand demi-axe, un petit demi-axe et un angle de rotation de l'ellipse (22a, 22b, 22c, 22d, 22e, 22f, 22g, 22h) à partir des au moins trois distances limites g dépendantes de l'angle d'orientation.

12. Système selon la revendication 10 ou 11, dans lequel le terminal (2) est un appareil mobile de traitement de données et de communication.

13. Système selon l'une des revendications 10 à 12, dans lequel l'unité de calcul est conçue pour exécuter un logiciel pour la mise en œuvre d'un procédé selon l'une des revendications 1 à 9, ce logiciel étant stocké dans une mémoire du terminal (2) et/ou devant être fourni par un serveur.

14. Application pour un terminal (2) de détermination d'au moins une caractéristique optométrique d'au moins un œil (8) d'une personne (6), le terminal (2) présentant un écran (4), l'application étant mise en œuvre sur le terminal (2), fonctionnant sur le terminal (2) et étant conçue pour afficher des signes visuels sensibles à l'orientation à l'écran (4), chaque signe visuel sensible à l'orientation présentant deux points en tant que repères, une propriété géométrique du signe visuel étant dépendante d'un angle d'orientation des deux repères et d'une distance de repérage entre les deux repères ou formée par celui-ci, l'application étant conçue pour déterminer pour au moins trois angles d'orientation différents à chaque fois un groupe de signes visuels sensibles à l'orientation, les repères des signes visuels sensibles à l'orientation de chaque groupe présentant des distances de repérage différentes d'un signe visuel à l'autre pour un angle d'orientation, l'application étant conçue pour afficher successivement à l'écran (4) les signes visuels sensibles à l'orientation pour chaque angle d'orientation, en commençant, pour chaque angle d'orientation, par un signe visuel sensible à l'orientation pour lequel la distance de repérage respective entre les repères présente une valeur définie, et pour afficher ensuite successivement d'autres signes visuels sensibles à l'orientation avec à chaque fois une autre distance de repérage entre des repères, l'application étant conçue pour vérifier quelle distance de repérage minimale entre les repères est encore reconnaissable par la personne (6) pour l'angle d'orientation respectif, le terminal (2) présentant au moins un module de sortie et au moins un module d'entrée, l'au moins un module de sortie étant conçu pour demander à la personne (6) si, pour un signe visuel sensible à l'orientation respectivement affiché, elle reconnaît encore ou non la distance de repérage entre les repères pour l'angle d'orientation respectif, l'au moins un module d'entrée étant conçu pour enregistrer une réponse respective qui est introduite par la personne (6) en actionnant l'au moins un module d'entrée, l'application étant conçue pour déterminer la distance de repérage minimale en tant que distance limite g pour chaque angle d'orientation, une fonction caractéristique pour l'au moins un œil (8) étant déduite par le terminal (2) à partir d'une pluralité de distances limites g, la fonction caractéristique décrivant une fonction de point zéro d'une fonction de transfert de modulation MTF dans un système de coordonnées virtuel, les points zéro de la fonction de transfert de modulation MTF décrivant une limite de résolution de l'œil (8), les repères, réalisés sous la forme de points, du signe visuel d'une fréquence limite et donc de la distance limite g dans l'image du signe visuel ne fournissant aucun contraste, la fréquence limite étant calculée à chaque fois à partir de l'inverse de la distance limite correspondante dépendante de l'angle d'orientation.

Fig. 1

Fig. 2 a

Fig. 2 b

Fig. 2 c

Fig. 2 d

Fig. 2 e

Fig. 2 f

Fig. 2 g

Fig. 3

Fig. 4

Fig. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2008018858 A1

- US 2021157168 A1

**In der Beschreibung aufgeführte Nicht-Patentliteratur**

- Resolving the clinical acuity categories "hand motion" and "counting fingers" using the Freiburg Visual Acuity Test (FrACT). **LANGE C et al.** GRAEFE'S ARCHIVE FOR CLINICAL AND EXPERIMENTAL OPHTHALMOLOGY; INCORPORATING GERMAN JOURNAL OF OPHTHALMOLOGY. SPRINGER, 03 September 2008, vol. 247, 137-142

- **WATSON** ; **ANDREW**. Computing human optical point spread functions. *Journal of vision.*, 2015, vol. 15